# EUROPÄISCHE PATENTSCHRIFT

(11) **EP 1 898 721 B1**
(45) Veröffentlichungstag und Bekanntmachung des Hinweises auf die Patenterteilung: **17.02.2010**
(21) Anmeldenummer: 06763923.7
(22) Anmeldetag: 28.06.2006
(51) Int. Cl.: A23L 1/275, A23L 1/303, A61K 31/015, C07C 403/24, C09B 61/00

(54) **VERFAHREN ZUR HERSTELLUNG EINER WÄSSRIGEN SUSPENSION UND EINER PULVERFÖRMIGEN ZUBEREITUNG EINES ODER MEHRERER CAROTINOIDE**
METHOD FOR PRODUCING AN AQUEOUS SUSPENSION AND A POWDERED PREPARATION OF ONE OR MORE CAROTINOIDS
PROCÉDÉ DE PRODUCTION D'UNE SUSPENSION AQUEUSE ET D'UNE PRÉPARATION PULVÉRULENTE D'UN OU DE PLUSIEURS CAROTÉNOÏDES

(30) Priorität: 30.06.2005 DE 102005030952
(43) Veröffentlichungstag der Anmeldung: 19.03.2008
(73) Patentinhaber: BASF SE, 67056 Ludwigshafen (DE)
(72) Erfinder: MUSAEUS, Nina, DK-2900 Hellerup (DK); JENSEN, Carsten, Ninn, DK-2750 Ballerup (DK)
(86) Internationale Anmeldenummer: PCT/EP2006/063627
(87) Internationale Veröffentlichungsnummer: WO 2007/003543

(56) Entgegenhaltungen:
- WO-A-91/06292
- WO-A-2006/032399
- US-A- 3 998 753
- US-A1- 2003 148 099
- PATENT ABSTRACTS OF JAPAN Bd. 006, Nr. 063 (C-099), 22. April 1982 (1982-04-22) & JP 57 003861 A (SUMITOMO CHEM CO LTD), 9. Januar 1982 (1982-01-09)
- MANZ U: "DIE ANWENDUNG UND BEDEUTUNG VON SYNTHETISCHEN CAROTINOIDEN IN DER LEBENS- UND FUTTERMITTELSOWIE IN DER PHARMAZEUTISCHEN INDUSTRIE" Juli 1967 (1967-07), CHIMIA, AARAU, CH, PAGE(S) 329-335 , XP009062672 ISSN: 0009-4293 in der Anmeldung erwähnt das ganze Dokument

## Beschreibung

Die Erfindung betrifft ein Verfahren zur Herstellung einer wässrigen Suspension und einer pulverförmigen Zubereitung eines oder mehrerer Carotinoide, insbesondere von Carotinoiden, ausgewählt aus der Gruppe, bestehend aus β-Carotin, Lutein, Zeaxanthin und Lycopin oder Mischungen davon.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Carbonylgruppen vor. Typische Vertreter dieser Gruppe sind u.a. Astaxanthin, Canthaxanthin, Lutein und Zeaxanthin.

Zu den Sauerstoff-haltigen Carotinoiden zählen auch Citranaxanthin und β-Apo-8'-Carotinsäureethylester.

Sauerstoff-haltige Carotinoide sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese sowohl synthetisch zugänglichen als auch aus natürlichen Quellen isolierbaren Polyene stellen für die Lebens- und Futtermittelindustrie sowie für den pharmazeutischen Bereich wichtige Farb- und Wirkstoffe dar und sind, wie im Falle von Astaxanthin, Wirkstoffe mit Provitamin-A Aktivität beim Lachs.

Sowohl Carotine als auch Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der durch chemische Synthese erhaltenen, relativ grobkörnigen Produkte in der Einfärbung von Lebens- und Futtermitteln entgegen, da die Substanzen in grobkristalliner Form nicht lagerstabil und nur schlechte Färbungsergebnisse liefern. Diese für die praktische Verwendung der Carotinoide nachteiligen Effekte wirken sich insbesondere im wässrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Carotinoide bzw. Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 µm.

Daneben existieren eine Reihe von kombinierten Emulgier-/Sprühtrocknungsverfahren, wie sie z. B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, dass man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wässrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

DE-A-44 24 085 beschreibt die Verwendung von teilabgebauten Sojaproteinen als Schutzkolloide für fettlösliche Wirkstoffe. Die hier offenbarten Sojaproteine weisen einen Abbaugrad von 0,1 bis 5 % auf.

In der deutschen Offenlegungsschrift DE-A-101 04 494 wird die Herstellung von Carotinoid Trockenpulvern unter Verwendung von Sojaproteinen zusammen mit Lactose als Schutzkolloide beschrieben.

US-A-2003 014 8099 offenbart eine Mikrokapsel zur Verwendung in Lebensmittel und Pflegeprodukte mit einer Schutzhülle, welche bevorzugt Saccharose und modifizierte Stärke (Marke Capsul) enthält. Die Gewichtsverhältnisse und Mengen an jeweiligen Komponenten fallen innerhalb der in den genannten Patentansprüche beanspruchten Bereiche. Die Mikrokapsel setzt Carotinoide nach der Einnahme frei.

US-A-3 998 753 offenbart ein Verfahren zur Herstellung von wasserdispergierbaren, carotinoidhaltigen Pulvermischungen, enthaltend Stabilisatore und Zusatzstoffe, z.B. Saccharose und modifizierte Stärke, bevorzugt Octenyl-Succinat Stärke.

Trotz der im eingangs genannten Stand der Technik bereits zahlreich beschriebenen Carotinoid-Formulierungen besteht weiterhin Bedarf nach Verbesserungen dieser Zubereitungen, sei es im Hinblick auf eine erhöhte Bioverfügbarkeit oder insbesondere im Hinblick auf eine bessere Lagerstabilität, beispielsweise in Tabletten.

Aufgabe der vorliegenden Erfindung war es daher, Carotinoid-haltige Zubereitungen vorzuschlagen, die die oben genannten Anforderungen erfüllen.

Diese Aufgabe wurde erfindungsgemäß gelöst mit einer wässrigen Carotinoid-haltigen Suspension, enthaltend
a) mindestens ein Carotinoid,
b) mindestens eine modifizierte Stärke und
c) Saccharose,
dadurch gekennzeichnet, dass die Suspension, bezogen auf die Trockenmasse der wässrigen Suspension, 1 bis 15 Gew.-%, bevorzugt 5 bis 15 Gew.-%, ganz besonders bevorzugt 8 bis 13 Gew.-% mindestens eines Carotinoids enthält und das Gewichtsverhältnis von Carotinoid a) zu Saccharose c) 1 zu 5 bis 1 zu 10 beträgt.

Als Carotinoide kommen im Rahmen der vorliegenden Erfindung u.a. α- und β-Carotin, Lycopin, Lutein, Astaxanthin, Zeaxanthin, Capsanthin, Capsorubin, α- und β-Cryptoxanthin, Citranaxanthin, Canthaxanthin, Bixin, β-Apo-4-carotinal, β-Apo-8-carotinal und β-Apo-8-carotinsäureester oder Mischungen davon in Frage. Bevorzugte Carotinoide sind β-Carotin, β-Cryptoxanthin, Lycopin, Lutein, Astaxanthin, Zeaxanthin und Canthaxanthin. Besonders bevorzugt sind Carotinoide, ausgewählt aus der Gruppe, bestehend aus β-Carotin, Lutein, Zeaxanthin und Lycopin oder Mischungen davon, ganz besonders bevorzugt sind β-Carotin, Lycopin und Lutein oder Mischungen davon, insbesondere β-Carotin.

Unter modifizierte Stärke sind chemisch oder enzymatisch hergestellte Umwandlungsprodukte der Stärke zu verstehen. Dabei kann es sich um Stärkeether, Stärkeester oder Stärkephosphate handeln. Bevorzugte Vertreter aus dieser Gruppe sind Stärkeester, insbesondere Octenylsuccinat Stärke z.B. Capsul^{®} (Natriumoctenylsuccinat Stärke) der Fa. National Starch.

Der Anteil an modifizierter Stärke beträgt, bezogen auf die Trockenmasse der wässrigen Suspension, 5 bis 50 Gew.-%, bevorzugt 5 bis 30 Gew.-%, besonders bevorzugt 8 bis 26 Gew.-%.

Eine bevorzugte Ausführungsform der erfindungsgemäßen wässrigen Suspension ist dadurch gekennzeichnet, dass sie mindestens ein Carotinoid als nanopartikuläre Teilchen enthält.

Als nanopartikuläre Teilchen sind solche Teilchen zu verstehen, die eine über Fraunhofer Beugung ermittelte mittlere Partikelgröße D[4,3] von 0,02 bis 100 µm, bevorzugt 0,05 bis 50 µm, besonders bevorzugt 0,05 bis 20 µm, ganz besonders bevorzugt 0,05 bis 5 µm, insbesondere 0,05 bis 1,0 µm aufweisen. Der Begriff D[4,3] bezeichnet den volumengewichteten mittleren Durchmesser (siehe Handbuch zu Malvern Mastersizer S, Malvern Instruments Ltd., UK).

Eine weiter bevorzugte Ausführungsform der erfindungsgemäßen wässrigen Suspension ist dadurch gekennzeichnet, dass sie als Komponente d) zusätzlich 0,1 bis 10 Gew.-%, bevorzugt 0,5 bis 5 Gew.-%, besonders bevorzugt 0,8 bis 2,5 Gew.-%, ganz besonders bevorzugt 1 bis 2 Gew.-% eines oder mehrerer Antioxidantien, bezogen auf die Trockenmasse der wässrigen Suspension, enthält. Beispiele für geeignete Antioxidantien sind u.a. α-Tocopherol, t-Butyl-hydroxy-toluol, t-Butylhydroxyanisol, Zitronensäure, Natriumcitrat, Ascorbinsäure, Natriumascorbat, Ascorbylpalmitat oder Ethoxyquin oder Mischungen davon. Bevorzugte Antioxidantien sind α-Tocopherol, Ascorbinsäure, Natriumascorbat, Ascorbylpalmitat oder Mischungen davon.

Zur Erhöhung der Stabilität des Wirkstoffes gegen mikrobiellem Abbau kann es zweckmäßig sein, der Zubereitung Konservierungsmittel wie z.B. Methyl-4-hydroxybenzoat, Propyl-4-hydroxybenzoat, Sorbinsäure oder Benzoesäure oder deren Salze zuzusetzen.

Die erfindungsgemäße wässrige Suspension enthält einen Feststoffanteil von 10 bis 80 Gew.-%, bevorzugt von 30 bis 75 Gew.-%, besonders bevorzugt von 50 bis 75 Gew.-%.

Neben der modifizierten Stärke können die erfindungsgemäßen wässrigen Suspensionen und die daraus hergestellten pulverförmigen Zubereitungen weitere Schutzkolloide enthalten. Dafür kommen beispielsweise die folgenden Stoffe in Frage:
Rinder-, Schweine- oder Fischgelatine, insbesondere sauer oder basisch abgebaute Gelatine mit Bloom-Zahlen im Bereich von 0 bis 250, ganz besonders bevorzugt Gelatine A 100, A 200, A 240, B 100 und B 200 sowie niedermolekulare, enzymatisch abgebaute Gelatinetypen mit der Bloom-Zahl 0 und Molekulargewichten von 15.000 bis 25.000 D wie zum Beispiel Collagel A und Gelitasol P (Firma Stoess, Eberbach) sowie Mischungen dieser Gelatine-Sorten.
Stärke, Dextrin, Pektin, Gummiarabicum, Ligninsulfonate, Chitosan, Polystyrolsulfonat, Alginate, Casein, Caseinat, Methylcellulose, Carboxymethylcellulose, Hydroxypropylcellulose oder Mischungen dieser Schutzkolloide.
Pflanzenproteine wie Soja-, Reis- und/oder Weizenproteine, wobei diese Pflanzenproteine teilabgebaut oder in nicht abgebauter Form vorliegen können.

Gegenstand der Erfindung ist auch ein Verfahren zur Herstellung einer wässrigen Carotinoid-haltigen Suspension dadurch gekennzeichnet, dass man
i) ein oder mehrere Carotinoide a) in einer wässrigen molekulardispersen oder kolloiddispersen Lösung von mindestens einer modifizierten Stärke b) und Saccharose c) suspendiert und
ii) die suspendierten Teilchen zerkleinert,
wobei die Suspension nach dem Verfahrensschritt ii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt

Gegenstand der Erfindung ist ebenfalls ein Verfahren zur Herstellung einer wässrigen Carotinoid-haltigen Suspension dadurch gekennzeichnet, dass man
i) ein oder mehrere Carotinoide a) in eine wässrige molekulardisperse oder kolloiddisperse Lösung von modifizierter Stärke b) suspendiert,
ii) die suspendierten Teilchen zerkleinert und
iii) die feinteilige Suspension mit Saccharose c) mischt,
wobei die Suspension nach dem Verfahrensschritt iii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt.

Der Wirkstoff [ein oder mehrere Carotinoide] wird dabei vor dem Zerkleinern bevorzugt in kristalliner Form in die oben genannte Schutzkolloidlösung suspendiert.

Das Zerkleinern der suspendierten Teilchen kann im Rahmen der vorliegenden Erfindung u.a. mittels eines Hochdruckhomogenisators oder bevorzugt durch Mahlung erfolgen.

Die Mahlung in den Verfahrensschritten ii) kann dabei in an sich bekannter Weise z.B. mit einer Kugelmühle erfolgen. Dabei wird je nach verwendetem Mühlentyp so lange gemahlen, bis die Teilchen eine Ober Fraunhofer Beugung ermittelte mittlere Partikelgröße D[4,3] von 0,02 bis 100 µm, bevorzugt 0,05 bis 50 µm, besonders bevorzugt 0,05 bis 20 µm, ganz besonders bevorzugt 0,05 bis 5 µm, insbesondere 0,05 bis 1,0 µm aufweisen. Der Begriff D[4,3] bezeichnet den vofumengewichteten mittleren Durchmesser (siehe Handbuch zu Malvern Mastersizer S, Malvern Instruments Ltd., UK).

Nähere Einzelheiten zur Mahlung und den dafür verwendeten Apparaturen finden sich u.a. in Ullmann's Encyclopedia of Industrial Chemistry, Sixth Edition, 2000, Electronic Release, Size Reduction, Kapitel 3.6.: Wet Grinding sowie in EP-A-0 498 824.

Der Kristallinitätsgrad der gemahlenen Carotinoid-Teilchen liegt nach dem Mahlprozess bei größer 80%, bevorzugt bei größer 90%, besonders bevorzugt bei größer 98%.

Bevorzugte Ausführungsformen hinsichtlich der Komponenten a) bis c) und ihren Einsatzmengen finden sich in den bereits eingangs gemachten Erläuterungen.

Eine besonders bevorzugte Ausführungsform des erfindungsgemäßen Verfahrens zur Herstellung einer wässrigen Carotinoid-haltigen Suspension ist dadurch gekennzeichnet, dass man
i) ein oder mehrere Carotinoide a), ausgewählt aus der Gruppe, bestehend aus β-Carotin, Lutein, Zeaxanthin und Lycopin oder Mischungen davon in eine wässrige molekulardisperse oder kolloiddisperse Lösung von Natriumoctenylsuccinat Stärke b) suspendiert,
ii) die suspendierten Teilchen mahlt und
iii) die gemahlene Suspension mit Saccharose c) mischt,
wobei die Suspension nach dem Verfahrensschritt iii), bezogen auf die Trockenmasse der wässrigen Suspension, 8 bis 13 Gew.-% mindestens eines Carotinoids a), ausgewählt aus der Gruppe, bestehend aus β-Carotin, Lutein, Zeaxanthin und Lycopin oder Mischungen davon enthält und das Gewichtsverhältnis von Carotinoid a) zu Saccharose c) 1 zu 5 bis 1 zu 10 beträgt.

Gegenstand der Erfindung ist weiterhin ein Verfahren zur Herstellung einer pulverförmigen Zubereitung, enthaltend mindestens ein Carotinoid, dadurch gekennzeichnet, dass man
i) ein oder mehrere Carotinoide a) in einer wässrigen molekulardispersen oder kolloiddispersen Lösung von mindestens einer modifizierten Stärke b) und Saccharose c) suspendiert,
ii) die suspendierten Teilchen mahlt und
iii) die Suspension anschließend, gegebenenfalls in Gegenwart eines Überzugsmaterials, in ein Trockenpulver überführt.
wobei die Suspension im Verfahrensschritt ii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt.

Gegenstand der Erfindung ist außerdem ein Verfahren zur Herstellung einer pulverförmigen Zubereitung, enthaltend mindestens ein Carotinoid, dadurch gekennzeichnet, dass man
i) ein oder mehrere Carotinoide a) in eine wässrige molekulardisperse oder kolloiddisperse Lösung von modifizierter Stärke b) suspensiert,
ii) die suspendierten Teilchen mahlt,
iii) die gemahlene Suspension mit Saccharose c) mischt und
iv) die Suspension anschließend gegebenenfalls in Gegenwart eines Überzugsmaterials in ein Trockenpulver überführt,
wobei die Suspension nach dem Verfahrensschritt iii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt.

Die Überführung in ein Trockenpulver kann dabei u.a. durch Sprühtrocknung, Sprühkühlung, modifizierte Sprühtrocknung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Nähere Einzelheiten zur Sprühkühlung und zur modifizierten Sprühtrocknung finden sich in WO 91/06292 (Seiten 5 bis 8).

Bevorzugte Ausführungsformen hinsichtlich der Komponenten a) bis c) und ihren Einsatzmengen finden sich in den bereits eingangs gemachten Erläuterungen.

Eine besonders bevorzugte Ausführungsform des erfindungsgemäßen Verfahrens zur Herstellung einer pulverförmigen Carotinoid-haltigen Zubereitung, ist dadurch gekennzeichnet, dass man
i) ein oder mehrere Carotinoide a), ausgewählt aus der Gruppe, bestehend aus β-Carotin, Lutein, Zeaxanthin und Lycopin oder Mischungen davon in eine wässrige molekulardisperse oder kolloiddisperse Lösung von Natriumoctenylsuccinat Stärke b) suspendiert,
ii) die suspendierten Teilchen mahlt und
iii) die gemahlene Suspension mit Saccharose c) mischt und,
iv) die Suspension anschließend gegebenenfalls in Gegenwart eines Überzugsmaterials in ein Trockenpulver überführt,
wobei die Suspension nach dem Verfahrensschritt iii), bezogen auf die Trockenmasse der wässrigen Suspension, 8 bis 13 Gew.-% mindestens eines Carotinoids a), ausgewählt aus der Gruppe, bestehend aus β-Carotin, Lutein, Zeaxanthin und Lycopin oder Mischungen davon enthält und das Gewichtsverhältnis von Carotinoid a) zu Saccharose c) 1 zu 5 bis 1 zu 10 beträgt.

Die Erfindung betrifft auch eine pulverförmige Zubereitung, enthaltend
a) mindestens ein Carotinoid, bevorzugt ausgewählt aus der Gruppe, bestehend aus β-Carotin, Lutein, Zeaxanthin und Lycopin oder Mischungen davon,
b) mindestens eine modifizierte Stärke, bevorzugt 5 bis 50 Gew.-%, besonders bevorzugt 5 bis 30 Gew.-%, ganz besonders bevorzugt 8 bis 26 Gew.-% Octenylsuccinat Stärke und
c) Saccharose,
dadurch gekennzeichnet, dass die Zubereitung, bezogen auf die Gesamtmasse der pulverförmigen Zubereitung, 1 bis 15 Gew.-%, bevorzugt 5 bis 15 Gew.-%, ganz besonders bevorzugt 8 bis 13 Gew.-% mindestens eines Carotinoids enthält und das Gewichtsverhältnis von Carotinoid a) zu Saccharose c) 1 zu 5 bis 1 zu 10 beträgt.

Eine weiter bevorzugte Ausführungsform der erfindungsgemäßen pulverförmigen Zubereitung ist dadurch gekennzeichnet, dass sie als Komponente d) zusätzlich 0,1 bis 10 Gew.-%, bevorzugt 0,5 bis 5 Gew.-%, besonders bevorzugt 0,8 bis 2,5 Gew.-%, ganz besonders bevorzugt 1 bis 2 Gew.-% eines oder mehrerer Antioxidantien, bezogen auf die Gesamtmasse der pulverförmigen Zubereitung, enthält.

Beispiele für geeignete Antioxidantien sind u.a. α-Tocopherol, t-Butyl-hydroxy-toluol, t-Butylhydroxyanisol, Zitronensäure, Natriumcitrat, Ascorbinsäure, Natriumascorbat, Ascorbylpalmitat oder Ethoxyquin oder Mischungen davon. Bevorzugte Antioxidantien sind α-Tocopherol, Ascorbinsäure, Natriumascorbat, Ascorbylpalmitat oder Mischungen davon.

Der Kristallinitätsgrad der in der pulverförmigen Zubereitung enthaltenen Carotinoid-Teilchen - beispielsweise bestimmbar durch Röntgenbeugungsmessungen - liegt bei größer 70%, bevorzugt im Bereich von 80 bis 100%, besonders bevorzugt im Bereich von 90 bis 99%, ganz besonders bevorzugt im Bereich von 95 bis 99%.

Die erfindungsgemäßen Trockenpulver zeichnen sich u.a. dadurch aus, dass sie sich in wässrigen Systemen unter Erzielung einer gleichmäßigen Feinverteilung des Wirkstoffes im Korngrößenbereich kleiner 1 µm problemlos wieder redispergieren lassen.

Die Verwendung der erfindungsgemäßen Kombination aus Saccharose und modifizierter Stärke als Forrnulierhilfsstoffe hat gegenüber anderen Zuckern, beispielsweise Lactose oder Glucose den Vorteil, dass die damit hergestellten Carotinoid-Formulierungen eine besonders hohe Lagerstabilität u.a. in Multivitamintabletten zeigen (siehe Tabelle).

Die erfindungsgemäßen Carotinoid-Formulierungen eignen sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich.

In den nachfolgenden Beispielen wird die Durchführung des erfindungsgemäßen Verfahrens näher erläutert.

### Beispiel 1

### Herstellung eines β-Carotin Trockenpulvers unter Verwendung einer Mischung aus Saccharose und Octenylsuccinatstärke (β-Carotin : Saccharose = 1 : 6,6)

a.
   Unter Schutzgas wurden 1160 g Wasser auf 55°C erwärmt und mit 26,5 g Natriumascorbat, 23,5 g Ascorbinsäure und 500 g Octenylsuccinatstärke (Capsul^{®}, Fa. National Starch) versetzt. In diese Lösung wurden unter Rühren 500 g kristallines β-Carotin einsuspendiert. Anschließend wurde die Suspension mit Hilfe einer Kugelmühle so lange gemahlen, bis die β-Carotinteilchen eine über Fraunhofer Beugung ermittelte mittlere Partikelgröße D[4,3] von ca. 0,6 µm aufwiesen.
b.
   2630 g dieser gemahlenen Suspension wurden unter Schutzgas in einen zweiten Reaktor überführt und unter Rühren mit 3207 g Saccharose und weiteren 469 g Octenylsuccinatstärke versetzt. Die Temperatur dieser Mischung wurde auf 55°C gehalten. Nach Zugabe von 30 g α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 11,1 % und hatte einen E1/1-Wert¹⁾ von 91.

### Beispiel 2

### Herstellung eines β-Carotin Trockenpulvers unter Verwendung einer Mischung aus sucrose und Octenylsuccinatstärke (β-Carotin : Saccharose = 1 : 7,2)

Unter Schutzgas werden 1160 g Wasser auf 55°C erwärmt und mit 26,5 g Natriumascorbat, 23,5 g Ascorbinsäure und 500 g Octenylsuccinatstärke (Capsul^{®}, Fa. National Starch) versetzt. In diese Lösung werden unter Rühren 500 g kristallines β-Carotin einsuspendiert und die Suspension wird mit Hilfe einer Kugelmühle so lange gemahlen, bis die β-Carotinteilchen eine über Fraunhofer Beugung ermittelte mittlere Partikelgröße D[4,3] von ca. 0,6 µm aufweisen.
b.
   2774 g dieser gemahlenen Suspension wurden unter Schutzgas in einen zweiten Reaktor überführt und unter Rühren mit 3914 g Saccharose versetzt. Die Temperatur dieser Mischung wurde auf 55°C gehalten. Nach Zugabe von 32 g α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 11,7 % und hatte einen E1/1-Wert¹⁾ von 89.

### Beispiel 3 (Vergleichsversuch)

### Herstellung eines β-Carotin Trockenpulvers unter Verwendung einer Mischung aus Glucosesirup und Octenylsuccinatstärke (β-Carotin : Glucosesirup =1 : 6,6)

a.
   Unter Schutzgas wurden 7,5 kg Wasser auf 55°C erwärmt und mit 0,225 kg Natriumascorbat, 0,2 kg Ascorbinsäure und 4,25 kg Octenylsuccinatstärke (Capsul^{®}, Fa. National Starch) versetzt. In diese Lösung wurden unter Rühren 4,25 kg kristallines β-Carotin einsuspendiert. Anschließend wurde die Suspension mit Hilfe einer Kugelmühle so lange gemahlen, bis die β-Carotinteilchen eine über Fraunhofer Beugung ermittelte mittlere Partikelgröße D[4,3] von ca. 0,6 µm aufwiesen.
b.
   2445 g dieser gemahlenen Suspension wurden unter Schutzgas in einen zweiten Reaktor überführt und unter Rühren mit 3590 g Glucosesirup und weiteren 419,7 g Octenylsuccinatstärke versetzt. Die Temperatur dieser Mischung wurde auf 55°C gehalten. Nach Zugabe von 26,9 g α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 10,9 % und hatte einen E1/1-Wert¹⁾ von 94.

### Beispiel 4 (Vergteichsversuch)

### Herstellung eines β-Carotin Trockenpulvers unter Verwendung einer Mischung aus Glucosesirup und Octenylsuccinatstärke (β-Carotin : Glucosesirup = 1 : 7,6)

2481 g der gemahlenen Suspension aus Beispiel 3a. wurden unter Schutzgas in einen zweiten Reaktor überführt und unter Rühren mit 4194 g Glucosesirup versetzt. Die Temperatur dieser Mischung wurde auf 55°C gehalten. Nach Zugabe von 27,4 g α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 10,6 % und hatte einen E1/1-Wert¹⁾ von 96.
¹⁾ Der E1/1-Wert definiert in diesem Zusammenhang die spezifische Extinktion einer 1%igen wässrigen Dispersion eines 10Gew.-%igen Trockenpulvers in einer 1 cm-Küvette im Absorptionsmaximum.

### Tabelle: Lagerstabilität der β-Carotin Beadlets in Multivitamin Tabletten

Die Stabilität der β-Carotin Beadlets wurde getestet anhand von Multivitamin-Mineral-Tabletten mit einem Gehalt von ca. 3 mg β-Carotin pro Tablette. Die Tabletten wurden in HDPE-Behältern verpackt, deren Deckel mit eingeschweister Aluminium Folie abgedichtet war. Die Lagerung der Tabletten erfolgte 6 Monate bei 40°C und 75% relativer Luftfeuchtigkeit. Der Gehalt an β-Carotin wurde jeweils nach 3 und 6 Monaten Lager zeit analysiert.

**Tabelle**

| | | | | | Nach 3 Monaten | | Nach 6 Monaten | |
|---|---|---|---|---|---|---|---|---|
| Bsp. | Zucker | Verhältnis BC: Zucker | Gehalt: β-Carotin | Anfangsgehalt β-Carotin pro Tablette [mg] | Gehalt β-Carotin pro Tablette [mg] | Verlust (%) | Gehalt β-Carotin pro Tablette [mg] | Verlust (%) |
| 1 | Saccharose | 1 : 6,6 | 11,1 | 3,66 | 3,19 | 12,8 | 3,00 | 18,0 |
| 2 | Saccharose | 1 : 7,2 | 11,6 | 3,86 | 3,39 | 12,2 | 3,46 | 10,4 |
| 3 | Glucosesirup | 1 : 6,6 | 10,9 | 3,60 | 2,67 | 25,8 | 2,75 | 23,6 |
| 4 | Glucosesirup | 1 : 7,6 | 10,6 | 3,40 | 2,57 | 24,4 | 2,45 | 27,9 |

## Patentansprüche

1. Wässrige Carotinoid-haltige Suspension, enthaltend
a) mindestens ein Carotinoid,
b) mindestens eine modifizierte Stärke und
c) Saccharose,
**dadurch gekennzeichnet, dass** die Suspension, bezogen auf die Trockenmasse der wässrigen Suspension, 1 bis 15 Gew.-% mindestens eines Carotinoids enthält und das Gewichtsverhältnis von Carotinoid a) zu Saccharose c) 1 zu 5 bis 1 zu 10 beträgt.

2. Wässrige Suspension nach Anspruch 1, **dadurch gekennzeichnet, dass** es sich bei der modifizierten Stärke b) um Octenyl-Succinat Stärke handelt.

3. Wässrige Suspension nach einem der Ansprüche 1 oder 2, **dadurch gekennzeichnet, dass** sie mindestens ein Carotinoid als nanopartikuläre Teilchen enthält.

4. Wässrige Suspension nach einem der Ansprüche 1 bis 3, enthaltend mindestens ein Carotinoid a), ausgewählt aus der Gruppe, bestehend aus β-Carotin, Lycopin, Zeaxanthin und Lutein oder Mischungen davon.

5. Wässrige Suspension nach einem der Ansprüche 1 bis 4, enthaltend als Komponente d) zusätzlich 0,1 bis 10 Gew.-% eines oder mehrerer Antioxidantien, bezogen auf die Trockenmasse der wässrigen Suspension.

6. Wässrige Suspension nach einem der Ansprüche 1 bis 5, enthaltend einen Feststoffanteil von 0,1 bis 80 Gew.-%.

7. Verfahren zur Herstellung einer wässrigen Carotinoid-haltigen Suspension **dadurch gekennzeichnet, dass** man
i) ein oder mehrere Carotinoide a) in einer wässrigen molekulardispersen oder kolloiddispersen Lösung von mindestens einer modifizierten Stärke b) und Saccharose c) suspendiert und
ii) die suspendierten Teilchen zerkleinert,
wobei die Suspension nach dem Verfahrensschritt ii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt.

8. Verfahren zur Herstellung einer wässrigen Carotinoid-haltigen Suspension **dadurch gekennzeichnet, dass** man
i) ein oder mehrere Carotinoide a) in eine wässrige molekulardisperse oder kolloiddisperse Lösung von modifizierter Stärke b) suspendiert,
ii) die suspendierten Teilchen zerkleinert und
iii) die feinteilige Suspension mit Saccharose c) mischt,
wobei die Suspension nach dem Verfahrensschritt iii), bezogen auf die Trockenmasse der wässrigen Suspension, 1 bis 25 Gew.-% mindestens eines Carotinoids enthält und das Gewichtsverhältnis von Carotinoide a) zu Saccharose c) 1 zu 2 bis 1 zu 80 beträgt.

9. Verfahren nach einem der Ansprüche 7 oder 8, **dadurch gekennzeichnet, dass** es sich bei der modifizierten Stärke b) um Octenyl-Succinat Stärke handelt.

10. Verfahren nach einem der Ansprüche 7 bis 9, **dadurch gekennzeichnet, dass** es sich bei den Carotinoiden a) um Verbindungen, ausgewählt aus der Gruppe, bestehend aus β-Carotin, Lycopin, Zeaxanthin und Lutein oder Mischungen davon handelt.

11. Verfahren zur Herstellung einer pulverförmigen Zubereitung, enthaltend mindestens ein Carotinoid, **dadurch gekennzeichnet, dass** man
i) ein oder mehrere Carotinoide a) in einer wässrigen molekulardispersen oder kolloiddispersen Lösung von mindestens einer modifizierten Stärke b) und Saccharose c) suspendiert,
ii) die suspendierten Teilchen zerkleinert und
iii) die Suspension anschließend, gegebenenfalls in Gegenwart eines Überzugsmaterials, in ein Trockenpulver überführt.
wobei die Suspension im Verfahrensschritt ii), bezogen auf die Trockenmasse der wässrigen Suspension, 1 bis 25 Gew.-% mindestens eines Carotinoids enthält und das Gewichtsverhältnis von Carotinoid a) zu Saccharose c) 1 zu 2 bis 1 zu 80 beträgt.

12. Verfahren zur Herstellung einer pulverförmigen Zubereitung, enthaltend mindestens ein Carotinoid, **dadurch gekennzeichnet, dass** man
i) ein oder mehrere Carotinoide a) in eine wässrige molekulardisperse oder kolloiddisperse Lösung von modifizierter Stärke b) suspensiert,
ii) die suspendierten Teilchen zerkleinert,
iii) die gemahlene Suspension mit Saccharose c) mischt und
iv) die Suspension anschließend gegebenenfalls in Gegenwart eines Überzugsmaterials in ein Trockenpulver überführt,
wobei die Suspension nach dem Verfahrensschritt iii), bezogen auf die Trockenmasse der wässrigen Suspension, 1 bis 25 Ges.-% mindestens eines Carotinoids enthält und das Gewichtsverhältnis von Carotinoid a) zu Saccharose c) 1 zu 2 bis 1 zu 80 beträgt.

13. Verfahren nach einem der Ansprüche 11 oder 12, **dadurch gekennzeichnet, dass** es sich bei den Carotinoiden a) um Verbindungen, ausgewählt aus der Gruppe, bestehend aus β-Carotin, Lycopin, Zeaxanthin und Lutein oder Mischungen davon handelt.

14. Verfahren nach einem der Ansprüche 11 bis 13, **dadurch gekennzeichnet, dass** es sich bei der modifizierten Stärke b) um Octenyl-Succinat Stärke handelt.

15. Pulverförmige Zubereitung, enthaltend
a) mindestens ein Carotinoid,
b) mindestens eine modifizierte Stärke und
c) Saccharose,
**dadurch gekennzeichnet, dass** die Zubereitung, bezogen auf die Gesamtmasse der pulverförmigen Zubereitung, 1 bis 15 Gew.-% mindestens eines Carotinoids enthält und das Gewichtsverhältnis von Carotinoid a) zu Saccharose c) 1 zu 5 bis 1 zu 10 beträgt.

16. Pulverförmige Zubereitung nach Anspruch 15, **dadurch gekennzeichnet, dass** es sich bei der modifizierten Stärke b) um Octenyl-Succinat Stärke handelt.

17. Pulverförmige Zubereitung nach einem der Ansprüche 15 oder 16, enthaltend mindestens ein Carotinoid a), ausgewählt aus der Gruppe, bestehend aus β-Carotin, Lycopin, Zeaxanthin und Lutein oder Mischungen davon.

18. Pulverförmige Zubereitung nach einem der Ansprüche 15 bis 17, enthaltend als Komponente d) zusätzlich 0,1 bis 10 Gew.-% eines Antioxidans, bezogen auf die Gesamtmasse der pulverförmigen Zubereitung.

19. Verwendung der wässrigen Suspension, definiert gemäß einem der Ansprüche 1 bis 6 als Zusatz zu Lebensmitteln, Nahrungsergänzungsmitteln, Tierfuttermitteln, pharmazeutischen und kosmetischen Zubereitungen.

20. Verwendung der pulverförmige Zubereitung, definiert gemäß einem der Ansprüche 15 bis 18 als Zusatz zu Lebensmitteln, Nahrungsergänzungsmitteln, Tierfuttermitteln, pharmazeutischen und kosmetischen Zubereitungen.

## Claims

1. An aqueous carotenoid-containing suspension comprising
a) at least one carotenoid,
b) at least one modified starch and
c) sucrose,
wherein the suspension comprises, based on the dry matter of the aqueous suspension, from 1 to 15% by weight of at least one carotenoid, and the ratio of carotenoid a) to sucrose c) is from 1:5 to 1:10 by weight.

2. The aqueous suspension according to claim 1, wherein the modified starch b) is octenylsuccinate starch.

3. The aqueous suspension according to either of claims 1 or 2, which comprises at least one carotenoid as nanoparticles.

4. The aqueous suspension according to any of claims 1 to 3, comprising at least one carotenoid a) selected from the group consisting of β-carotene, lycopene, zeaxanthin and lutein or mixtures thereof.

5. The aqueous suspension according to any of claims 1 to 4, comprising as component d) in addition from 0.1 to 10% by weight of one or more antioxidants, based on the dry matter of the aqueous suspension.

6. The aqueous suspension according to any of claims 1 to 5, comprising a solids content of from 0.1 to 80% by weight.

7. A process for producing an aqueous carotenoid-containing suspension, which comprises
i) suspending one or more carotenoids a) in an aqueous molecular dispersed or colloidal solution of at least one modified starch b) and sucrose c), and
ii) comminuting the suspended particles,
where the suspension comprises after process step ii), based on the dry matter of the aqueous suspension, from 1 to 25% by weight of at least one carotenoid, and the ratio of carotenoid a) to sucrose c) is from 1:2 to 1:80 by weight.

8. A process for producing an aqueous carotenoid-containing suspension, which comprises
i) suspending one or more carotenoids a) in an aqueous molecular dispersed or colloidal solution of modified starch b),
ii) comminuting the suspended particles, and
iii) mixing the fine-particle suspension with sucrose c),
where the suspension comprises after process step iii), based on the dry matter of the aqueous suspension, from 1 to 25% by weight of at least one carotenoid, and the ratio of carotenoid a) to sucrose c) is from 1:2 to 1:80 by weight.

9. The process according to either of claims 7 or 8, wherein the modified starch b) is octenylsuccinate starch.

10. The process according to any of claims 7 to 9, wherein the carotenoids a) are compounds selected from the group consisting of β-carotene, lycopene, zeaxanthin and lutein or mixtures thereof.

11. A process for producing a preparation in powder form, comprising at least one carotenoid, which comprises
i) suspending one or more carotenoids a) in an aqueous molecular dispersed or colloidal solution of at least one modified starch b) and sucrose c),
ii) comminuting the suspended particles, and
iii) subsequently converting the suspension if appropriate in the presence of a coating material into a dry powder,
where the suspension comprises in process step ii), based on the dry matter of the aqueous suspension, from 1 to 25% by weight of at least one carotenoid, and the ratio of carotenoid a) to sucrose c) is from 1:2 to 1:80 by weight.

12. A process for producing a preparation in powder form, comprising at least one carotenoid, which comprises
i) suspending one or more carotenoids a) in an aqueous molecular dispersed or colloidal solution of modified starch b),
ii) comminuting the suspended particles,
iii) mixing the ground suspension with sucrose c), and
iv) subsequently converting the suspension if appropriate in the presence of a coating material into a dry powder,
where the suspension comprises after process step iii), based on the dry matter of the aqueous suspension, from 1 to 25% by weight of at least one carotenoid, and the ratio of carotenoid a) to sucrose c) is from 1:2 to 1:80 by weight.

13. The process according to either of claims 11 or 12, wherein the carotenoids a) are compounds selected from the group consisting of β-carotene, lycopene, zeaxanthin and lutein or mixtures thereof.

14. The process according to any of claims 11 to 13, wherein the modified starch b) is octenylsuccinate starch.

15. A preparation in powder form, comprising
a) at least one carotenoid,
b) at least one modified starch and
c) sucrose,
wherein the preparation comprises, based on the total mass of the preparation in powder form, from 1 to 15% by weight of at least one carotenoid, and the ratio of carotenoid a) to sucrose c) is from 1:5 to 1:10 by weight.

16. The preparation in powder form according to claim 15, wherein the modified starch b) is octenylsuccinate starch.

17. The preparation in powder form according to either of claims 15 or 16, comprising at least one carotenoid a) selected from the group consisting of β-carotene, lycopene, zeaxanthin and lutein or mixtures thereof.

18. The preparation in powder form according to any of claims 15 to 17, comprising as component d) in addition from 0.1 to 10% by weight of an antioxidant, based on the total mass of the preparation in powder form.

19. The use of the aqueous suspension defined in any of claims 1 to 6 as addition to human foods, dietary supplements, animal feeds, pharmaceutical and cosmetic preparations.

20. The use of the preparation in powder form defined in any of claims 15 to 18 as addition to human foods, dietary supplements, animal feeds, pharmaceutical and cosmetic preparations.

## Revendications

1. Suspension aqueuse contenant un/des caroténoïde(s), contenant
a) au moins un caroténoïde,
b) au moins un amidon modifié et
c) du saccharose,
**caractérisée en ce que** la suspension, par rapport à la masse sèche de la suspension aqueuse, contient 1 à 15% en poids d'au moins un caroténoïde et le rapport pondéral de caroténoïde a) à saccharose c) est de 1:5 à 1:10.

2. Suspension aqueuse selon la revendication 1, **caractérisée en ce qu'**il s'agit, pour l'amidon modifié b), d'octényl-succinate-amidon.

3. Suspension aqueuse selon l'une quelconque des revendications 1 ou 2, **caractérisée en ce qu'**elle contient au moins un caroténoïde sous forme de particules nanoparticulaires.

4. Suspension aqueuse selon l'une quelconque des revendications 1 à 3, contenant au moins un caroténoïde a), choisi dans le groupe constitué par le β-carotène, la lycopine, la zéaxanthine et la lutéine ou leurs mélanges.

5. Suspension aqueuse selon l'une quelconque des revendications 1 à 4, contenant comme composant d) en outre 0,1 à 10% en poids d'un ou de plusieurs antioxydants, par rapport à la masse sèche de la suspension aqueuse.

6. Suspension aqueuse selon l'une quelconque des revendications 1 à 5, contenant une proportion de solides de 0,1 à 80% en poids.

7. Procédé pour la préparation d'une suspension aqueuse contenant un/des caroténoïde(s), **caractérisé en ce qu'**on
i) met en suspension un ou plusieurs caroténoïdes a) dans une suspension aqueuse à dispersion moléculaire ou à dispersion colloïdale d'au moins un amidon modifié b) et de saccharose c) et
ii) broie les particules en suspension,
où la suspension, après l'étape de procédé ii), par rapport à la masse sèche de la suspension aqueuse, contient 1 à 25% en poids d'au moins un caroténoïde et le rapport pondéral de caroténoïde a) à saccharose c) est de 1:2 à 1:80.

8. Procédé pour la préparation d'une suspension aqueuse contenant un/des caroténoïde(s), **caractérisé en ce qu'**on
i) met en suspension un ou plusieurs caroténoïdes a) dans une solution aqueuse à dispersion moléculaire ou à dispersion colloïdale d'au moins un amidon modifié b) et
ii) broie les particules en suspension, et
iii) mélange la suspension finement divisée avec le saccharose c),
où la suspension, après l'étape de procédé iii), par rapport à la masse sèche de la suspension aqueuse, contient 1 à 25% en poids d'au moins un caroténoïde et le rapport pondéral de caroténoïde a) à saccharose c) est de 1:2 à 1:80.

9. Procédé selon l'une quelconque des revendications 7 ou 8, **caractérisé en ce qu'**il s'agit, pour l'amidon modifié b), d'octényl-succinate-amidon.

10. Procédé selon l'une quelconque des revendications 7 à 9, **caractérisé en ce qu'**il s'agit pour les caroténoïdes a) de composés choisis dans le groupe constitué par le β-carotène, la lycopine, la zéaxanthine et la lutéine ou leurs mélanges.

11. Procédé pour la préparation d'une composition poudreuse, contenant au moins un caroténoïde, **caractérisé en ce qu'**on
i) met en suspension un ou plusieurs caroténoïdes a) dans une suspension aqueuse à dispersion moléculaire ou à dispersion colloïdale d'au moins un amidon modifié b) et de saccharose c),
ii) broie les particules en suspension, et
iii) transforme ensuite la suspension, le cas échéant en présence d'un matériau d'enrobage, en une poudre sèche,
où la suspension, dans l'étape de procédé ii), par rapport à la masse sèche de la suspension aqueuse, contient 1 à 25% en poids d'au moins un caroténoïde et le rapport pondéral de caroténoïde a) à saccharose c) est de 1:2 à 1:80.

12. Procédé pour la préparation d'une composition poudreuse, contenant au moins un caroténoïde, **caractérisé en ce qu'**on
i) met en suspension un ou plusieurs caroténoïdes a) dans une solution aqueuse à dispersion moléculaire
ou à dispersion colloïdale d'au moins un amidon modifié b),
ii) broie les particules en suspension,
iii) mélange la suspension broyée avec le saccharose c) et
iv) transforme ensuite la suspension, le cas échéant en présence d'un matériau d'enrobage, en une poudre sèche,
où la suspension, après l'étape de procédé iii), par rapport à la masse sèche de la suspension aqueuse, contient 1 à 25% en poids d'au moins un caroténoïde et le rapport pondéral de caroténoïde a) à saccharose c) est de 1:2 à 1:80.

13. Procédé selon l'une quelconque des revendications 11 ou 12, **caractérisé en ce qu'**il s'agit pour les caroténoïdes a) de composés choisis dans le groupe constitué par le β-carotène, la lycopine, la zéaxanthine et la lutéine ou leurs mélanges.

14. Procédé selon l'une quelconque des revendications 11 à 13, **caractérisé en ce qu'**il s'agit, pour l'amidon modifié b), d'octényl-succinate-amidon.

15. Composition poudreuse, contenant
a) au moins un caroténoïde,
b) au moins un amidon modifié et
c) du saccharose,
**caractérisée en ce que** la suspension, par rapport à la masse totale de la composition poudreuse, contient 1 à 15% en poids d'au moins un caroténoïde et le rapport pondéral de caroténoïde a) à saccharose c) est de 1:5 à 1:10.

16. Composition poudreuse selon la revendication 15, **caractérisée en ce qu'**il s'agit, pour l'amidon modifié b), d'octényl-succinate-amidon.

17. Composition poudreuse selon l'une quelconque des revendications 15 ou 16, contenant au moins un caroténoïde a), choisi dans le groupe constitué par le β-carotène, la lycopine, la zéaxanthine et la lutéine ou leurs mélanges.

18. Composition poudreuse selon l'une quelconque des revendications 15 à 17, contenant comme composant d) en outre 0,1 à 10% en poids d'un antioxydant, par rapport à la masse totale de la composition poudreuse.

19. Utilisation de la suspension aqueuse, définie selon l'une quelconque des revendications 1 à 6, comme additif pour des aliments, des compléments alimentaires, des fourrages pour animaux, des préparations pharmaceutiques et cosmétiques.

20. Utilisation de la composition poudreuse, définie selon l'une quelconque des revendications 15 à 18, comme additif pour des aliments, des compléments alimentaires, des fourrages pour animaux, des préparations pharmaceutiques et cosmétiques.
